# FASCICULE DE BREVET EUROPEEN

(11) **EP 1 091 210 B1**
(45) Date de publication et mention de la délivrance du brevet: **24.03.2010**
(21) Numéro de dépôt: 00402489.9
(22) Date de dépôt: 08.09.2000
(51) Int. Cl.: G01N 33/24, G01N 31/12, G01N 25/14

(54) **Méthode d'analyse par pyrolyse**
Pyrolytisches Analyseverfahren
Analysing method using pyrolysis

(30) Priorité: 23.09.1999 FR 9911923
(43) Date de publication de la demande: 11.04.2001
(73) Titulaire: Institut Français du Pétrole, 92852 Rueil-Malmaison Cedex (FR)
(72) Inventeur: Haeseler, Franck, 92250 La Garenne Colombes (FR); Benoit, Yves, 95310 Saint Ouen L'Aumone (FR)

(56) Documents cités:
- EP-A- 0 590 932
- DE-A- 19 726 813
- GB-A- 2 161 269
- US-A- 4 352 673
- US-A- 5 355 736
- US-A- 5 698 774
- US-A- 5 786 225
- US-A- 5 922 974

## Description

La présente invention concerne une méthode pour conditionner un échantillon de roche, de sédiment ou de sol, afin de déterminer par pyrolyse au moins une caractéristique de pollution d'un sol naturel potentiellement ou réellement contaminé par des produits polluants, notamment des hydrocarbures et/ou dérivés. L'invention permet également l'analyse de produits polluants purs, par exemple des goudrons, des bitumes, etc.

Pour une meilleure compréhension, on rappelle que:
- au cours d'un déversement accidentel ou chronique (par exemple, rupture d'un pipe-line, perte d'étanchéité d'un stockage, par exemple une cuve ou une citerne, ou après abandon d'anciens sites industriels, une certaine quantité de composés hydrocarbonés peuvent s'infiltrer dans les sols, ce qui donne lieu à une pollution de tout ou partie dudit sol,
- la connaissance du type de polluant (essence, kérosène, gazole, lubrifiant, goudrons, bitume, dérivés chlorés,...) ainsi que la connaissance de l'extension dans l'espace et dans le temps de la pollution sont d'un grand intérêt pour les responsables chargés d'études de diagnostics, d'impact sur l'environnement et de la réhabilitation des sols pollués. Il est en effet connu que les techniques de réhabilitation employées dépendent du type de produits polluants.

De ce fait, il est très important de déterminer rapidement la nature des produits polluants et la quantité de sol à traiter qui dépend directement de l'extension de la pollution tant en profondeur qu'en surface. La détermination du degré de pollution d'un sol permet d'évaluer les volumes de terrain à traiter, de déterminer les meilleures méthodes de traitement, et ainsi les coûts correspondants à la mise en oeuvre.

Il apparaît alors que l'analyse systématique d'échantillons de sols potentiellement ou réellement pollués permet d'établir rapidement un diagnostic portant sur la nature et l'étendue de la pollution ainsi que sur les risques essentiels associés (contamination d'une nappe phréatique, par exemple).

Pour obtenir des analyses qualitatives et/ou quantitatives fiables, il est généralement nécessaire d'effectuer des pyrolyses sur des échantillons de produits polluants purs.

La connaissance de tels renseignements permet d'optimiser les opérations de réhabilitation des sites contaminés dès le stade du diagnostic. Sans les éliminer complètement, on limite les analyses de laboratoire longues et coûteuses, à un minimum nécessaire en complément des renseignements collectés systématiquement à l'aide de la méthode selon la présente invention.

On connaît la technique ROCK-EVAL développée par la demanderesse et décrite notamment dans les documents US-A-4153415, 4229181, 4352673, 4519983, ainsi que la demande de brevet française FR 94/08383. Cette méthode qui est rapide, pratiquement automatique, a été développée pour la caractérisation des roches mères ou des roches réservoirs, ainsi que des hydrocarbures qu'elles contiennent.

Cependant, cette méthode et le dispositif ne sont pas entièrement adaptés pour caractériser de façon précise les différentes coupes hydrocarbonées que peuvent contenir des sols pollués par de tels produits.

On connaît par le document FR-2753271 (US-5786225), un perfectionnement de la technique ROCK-EVAL permettant de caractériser de façon plus précise des polluants, notamment de type hydrocarbures et/ou dérivés (composés chlorés, soufrés,...), contenus dans un sol pollué.

Mais, lors d'une analyse de sols pollués, les fractions polluantes les plus légères, qui peuvent être apparentées à des coupes essence et/ou kérosène ayant moins de 12 atomes de carbones, ont la propriété d'être très volatils. Bien que les nacelles renfermant l'échantillon à analyser puissent être insérées dans un passeur d'échantillon refroidi, les pertes en composés volatils peuvent dépasser 90% en 24 heures. Ceci s'explique par les tensions de vapeurs élevées de ces composés légers, et le fait que les nacelles contenant les échantillons ne soient pas fermées hermétiquement.

En conséquence, la présente invention a pour objet de limiter ou de supprimer ces pertes par évaporation, ou d'agir sur la vitesse d'évaporation.

L'invention concerne une méthode d'analyse par pyrolyse sur un échantillon de sol ou de roche pollué par des hydrocarbures, ou d'un produit polluant à base d'hydrocarbures. On met dans un réceptacle l'échantillon et une quantité déterminée d'un matériau ayant la capacité d'adsorber au moins en partie les hydrocarbures. Un exemple d'utilisation d'un matériau adsorbant pour traiter des composts en réduisant la volatilité de rejets hydrocarburés est décrit dans le document US-A-5 685 891. On place le réceptacle dans un four de pyrolyse, et on effectue des mesures sur les effluents de pyrolyse provenant de l'échantillon et des hydrocarbures adsorbés.

L'échantillon peut être encadré dans le réceptacle par deux couches de matériau adsorbant.

L'échantillon peut être au moins partiellement mélangé avec le matériau adsorbant.

Le produit adsorbant peut être constitué par l'un au moins des matériaux suivants: silice, carbone graphitisé, bentone, tamis moléculaire, argile.

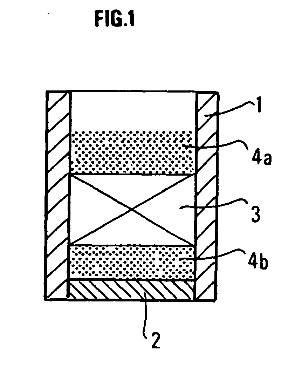

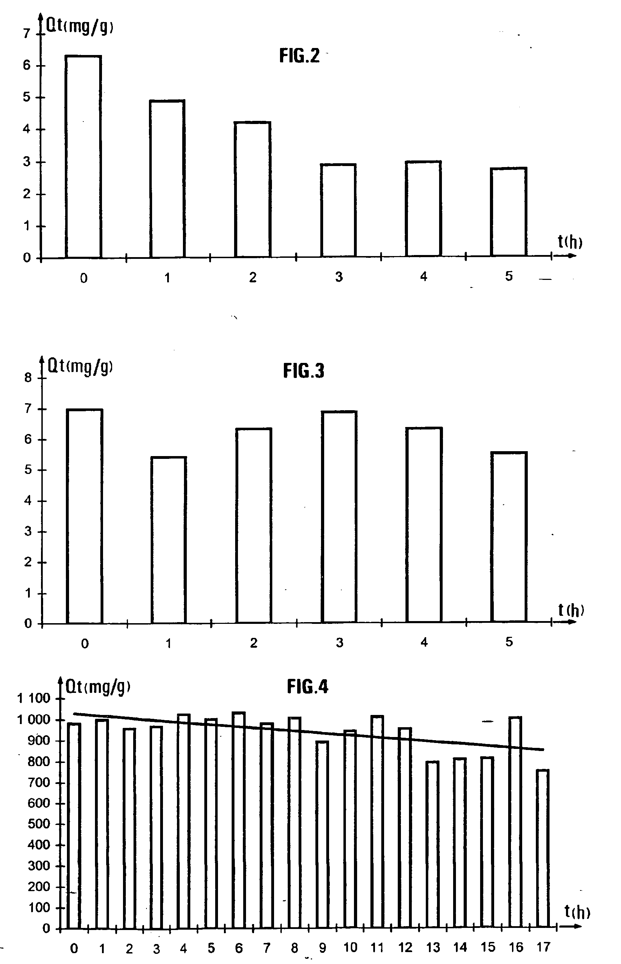

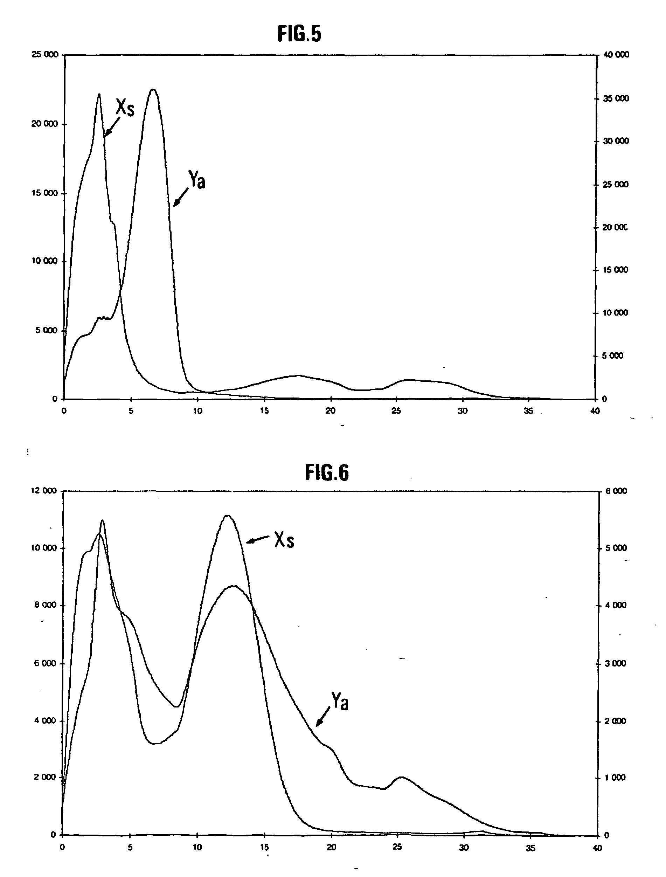

La présente invention sera mieux comprise et ses avantages apparaîtront plus clairement à la lecture de la description des exemples de réalisation, nullement limitatifs, illustrés par les figures ci-après annexées, parmi lesquelles:
■ La figure 1 montre en coupe une nacelle faisant office de réceptacle pour l'échantillon.
■ La figure 2 illustre une analyse effectuée avec un dispositif selon l'art antérieur.
■ La figure 3 illustre une analyse effectuée avec la méthode de la présente invention.
■ La figure 4 illustre une analyse similaire à la précédente, mais pendant une durée plus grande.
■ Les figures 5 et 6 montrent en comparaison l'analyse d'échantillons de deux roches différentes, avec ou sans silice.

Le dispositif de l'art antérieur, décrit dans le document FR-2753271 (US-5786225), cité ici en référence, comporte un système de refroidissement au niveau de la passerelle des nacelles d'échantillon, ou des nacelles étanches.

Le principe de la présente invention est fondé sur l'effet de l'addition d'un produit adsorbant, par exemple de la silice, au sein d'un échantillon de sol, ou de roche, pollué par une fraction légère d'hydrocarbure, le but étant de minimiser les pertes d'hydrocarbures légers par évaporation en adsorbant ces fractions légères, par exemple sur la silice.

La figure 1 montre en coupe une nacelle constituée par un cylindre métallique 1 partiellement obturé au fond par une pastille de métal fritté 2. Le fond en matériau fritté permet à un gaz vecteur de traverser la nacelle de façon à balayer les matériaux placés dans la nacelle. Le volume d'échantillon 3 est encadré par deux couches de silice 4a et 4b. Les dimensions de la nacelle cylindrique sont, de préférence: environ 7 à 8 mm de diamètre pour 18 mm de hauteur. Le poids d'échantillon de sol peut être compris entre 100 et 400 mg. Sans sortir de la présente invention, il pourrait y avoir un mélange entre le matériau adsorbant et l'échantillon de sol pollué.

### Essais:

- Six nacelles selon l'art antérieur sont remplies de manière identique d'un sol, homogénéisé, pollué par des hydrocarbures de type essence, enrichi artificiellement avec du kérosène (échantillon X). Ces nacelles sont placées simultanément sur le carrousel du dispositif tel qu'écrit dans le document FR-2753271 (US-5786225). Leur contenu est analysé à intervalle de temps t régulier (environ 1 heure). Le critère observé est la concentration totale Qt en hydrocarbures de l'échantillon (en mg/g). Cette valeur est représentative des fractions les plus légères, l'échantillon X ne contenant que de l'essence et du kérosène. Les résultats sont présentés sur la figure 2. Cette figure montre une perte d'hydrocarbures de l'ordre de 20% par heure. De ce fait, l'analyse dans l'appareil POLLUT-EVAL, n'est pas représentative d'un point de vue quantitatif. D'autre part, d'un point de vue qualitatif, cette perte peut conduire à une conclusion erronée quant à la source contaminante.
- Six nacelles, identiques aux précédentes, sont préparées avec le même échantillon X de sol pollué. L'échantillon est déposé sur une couche de silice puis recouvert par une autre couche de silice. Les nacelles sont placées simultanément sur le carrousel du dispositif de mesure. Le cycle de mesure est effectué selon l'essai précédent. La figure 3 montre l'effet de la rétention de la silice conduisant à une concentration en hydrocarbures stable dans le temps. Ces résultats montrent que l'ajout de la silice dans le réceptacle répond au problème posé par le maintien des hydrocarbures légers au sein de l'échantillon de sol.
- L'effet de la silice dans les nacelles a été étudié sur une période plus longue. Le même essai que précédemment a été effectué à partir de 17 nacelles comprenant de la silice, et dont le contenu est analysé à intervalle d'une heure. Les résultats montrés sur la figure 4 ne laissent apparaître pas, ou peu, de pertes pendant les 12 premières heures. La silice est une solution au problème de l'analyse des composés volatils.

L'ensemble de ces résultats montre que l'addition à un échantillon de sol pollué par des hydrocarbures d'un matériau susceptible d'adsorber et de piéger les hydrocarbures les plus volatils permet d'obtenir des analyses par pyrolyse optimisées.

Bien entendu, l'effet de la rétention par adsorption des hydrocarbures a une conséquence sur les profils des différents pics Q₀, Q₁, Q₂, Q₃,....mesurés par un appareil du type POLLUT-EVAL. Mais ces dérives peuvent être prises en compte par les programmes d'analyse permettant d'obtenir des résultats qualitatifs et quantitatifs.

Cet effet de rétention des hydrocarbures permet, entre autre, d'obtenir un profil de pyrogramme représentatif du produit polluant sans être perturbé par l'effet de matrice, c'est à dire la rétention plus ou moins grande des roches. La figure 5 montre deux pyrogrammes Xs et Ys (en abscisse le temps et en ordonnée la réponse des capteurs FID). Xs est un échantillon de sol sableux pollué, et Ya est un échantillon de sol argileux. A la lecture de ces deux pyrogrammes de la figure 5, on en déduirait que les deux échantillons sont pollués par des produits différents. Or, le même produit polluant a été utilisé pour préparer les échantillons Xs et Ya. Ce décalage provient de la différence de rétention des hydrocarbures légers entre le sol sableux et le sol argileux.

La figure 6 montre pour des mêmes échantillons Xs et Ya analysés selon la présente invention, c'est à dire en présence de produit adsorbant dans les nacelles, qu'il y a coïncidence des pyrogrammes, donc que les produits polluants des échantillons Xs et Ya sont les mêmes ou très proches. L'évaluation qualitative du polluant devient bien plus fondée avec la présente invention.

L'invention ne se limite pas à la silice comme adsorbant, on pourra utiliser des tamis moléculaires, des carbones graphitisés, de la bentone, des argiles, ou d'autres produits ayant la capacité d'adsorber des hydrocarbures ou dérivés.

L'invention ne se limite pas à des échantillons de sol ou de roche imprégnés de polluant, il est clair que l'on peut appliquer la présente invention à l'analyse de produit, plus ou moins purs, tels des goudrons, des bitumes.

## Revendications

1. Méthode d'analyse par pyrolyse sur un échantillon de sol ou de roche pollué par des hydrocarbures, ou d'un produit polluant à base d'hydrocarbures, **caractérisée en ce que** l'on effectue les étapes suivantes:
• on met dans un réceptacle ledit échantillon et une quantité déterminée d'un matériau ayant la capacité d'adsorber au moins en partie lesdits hydrocarbures,
• on place ledit réceptacle dans un four de pyrolyse,
• on effectue des mesures sur les effluents de pyrolyse provenant de l'échantillon et des hydrocarbures adsorbés.

2. Méthode selon la revendication 1, dans lequel on encadre ledit échantillon par deux couches de matériau adsorbant, lors de la mise dans ledit réceptacle.

3. Méthode selon la revendication 1, dans lequel on mélange au moins partiellement ledit échantillon avec ledit matériau adsorbant, lors de la mise dans ledit réceptacle.

4. Méthode selon l'une des revendications précédentes, dans lequel le produit adsorbant est constitué par l'un au moins des matériaux suivants: silice, carbone graphitisé, bentone, tamis moléculaire, argile.

## Claims

1. A method of analysis by pyrolysis of a sample of soil or rock polluted by hydrocarbons, or of a hydrocarbon-based pollutant product, **characterised in that** the following steps are carried out:
• placing said sample and a set quantity of a material, which can at least in part adsorb said hydrocarbons, in a receptacle,
• placing said receptacle in a pyrolytic oven,
• carrying out measurements on the pyrolysis effluents that come from the sample and the adsorbed hydrocarbons.

2. The method according to claim 1, in which said sample is surrounded in the receptacle by two adsorbent material layers, when being placed in said receptacle.

3. The method according to claim 1, in which said sample is at least partly mixed with said adsorbent material, when being placed in said receptacle.

4. The method according to one of the preceding claims, in which the adsorbent product is constituted by at least one of the following materials: silica, graphitised carbon, bentone, molecular sieve, clay.

## Patentansprüche

1. Verfahren zur Analyse durch Pyrolyse einer Boden- oder Gesteinsprobe, die durch Kohlenwasserstoffe oder durch einen Verschmutzungsstoff, der auf Kohlenwasserstoffen basiert, verschmutzt ist, **dadurch gekennzeichnet, dass** die folgenden Schritte durchgeführt werden:
- die Probe und eine bestimmte Menge eines Materials, das die Fähigkeit hat, mindestens teilweise die Kohlenwasserstoffe zu adsorbieren, werden in einen Behälter gegeben,
- der Behälter wird in einem Pyrolyseofen angeordnet,
- es werden Messungen an den Pyrolyseabflüssen, die aus der Probe stammen, und den adsorbierten Kohlenwasserstoffen durchgeführt.

2. Verfahren nach Anspruch 1, wobei die Probe, wenn sie in den Behälter gegeben wird, mit zwei Schichten des adsorbierenden Materials umgeben wird.

3. Verfahren nach Anspruch 1, wobei die Probe, wenn sie in den Behälter gegeben wird, mindestens teilweise mit dem adsorbierenden Material gemischt wird.

4. Verfahren nach einem der vorhergehenden Ansprüche, wobei das adsorbierende Produkt aus mindestens einem der folgenden Materialien besteht: Kieselerde, graphitierter Ruß, Benton, Molekularsieb, Ton.
